# FASCICULE DE BREVET EUROPEEN

(11) **EP 1 192 179 B1**
(45) Date de publication et mention de la délivrance du brevet: **09.11.2005**
(21) Numéro de dépôt: 00946023.9
(22) Date de dépôt: 27.06.2000
(51) Int. Cl.: C07K 7/06, C12N 9/18

(54) **COMPOSE PEPTIDIQUE DERIVE D'UNE ORF DECALEE DU GENE iCE**
PEPTIDVERBINDUNG, DIE VON EINEM VERSCHOBENEN OFFENEN LESERAHMEN DES ICE-GENS ABSTAMMT
PEPTIDE COMPOUND DERIVED FROM A FRAME-SHIFTED ORF OF THE ICE GENE

(30) Priorité: 28.06.1999 FR 9908224
(43) Date de publication de la demande: 03.04.2002
(73) Titulaire: Institut Gustave Roussy, 94800 Villejuif (FR)
(72) Inventeur: RONSIN, Christophe, F-92140 Clamart (FR); SCOTT, Véronique, F-94500 Champigny (FR); TRIEBEL, Frédéric, F-78000 Versailles (FR)
(74) Mandataire: Warcoin, Jacques
(86) Numéro de dépôt international: PCT/FR2000/001791
(87) Numéro de publication internationale: WO 2001/000784

(56) Documents cités:
- WO-A-97/29183
- WO-A-98/55133
- WO-A-99/18206
- DATABASE GENEMBL [en ligne] 28 avril 1997 (1997-04-28) SCHWER ET AL.: "H.sapiens mRNA for putative carboxylesterase" XP002135540 cité dans la demande -& SCHWER ET AL.: "Molecular cloning and characterization of a novel putative carboxylesterase, present in human instestine and liver" BIOCHEMICAL AND BIOPHYSICAL RESEARCH COMMUNICATIONS, vol. 233, no. 1, 1997, pages 117-120, XP000891817
- DATABASE GENEMBL [en ligne] 10 juillet 1996 (1996-07-10) PINDEL ET AL.: "Human carboxylesterase (hCE-2) mRNA, complete cds." XP002135541 -& PINDEL ET AL.: "Purification and cloning of a broad substrate specificity human liver carboxylestarase that catalyzes the hydrolysis of cocaine and heroin" JOURNAL OF BIOLOGICAL CHEMISTRY, vol. 272, no. 23, 6 juin 1997 (1997-06-06), pages 14769-14775, XP002135538
- DATABASE GENEMBL [en ligne] 25 novembre 1997 (1997-11-25) SONE,T.: "Homo sapiens mRNA for carboxylesterase, complete cds." XP002135542
- HERR WOLFGANG ET AL: "The use of computer-assisted video image analysis for the quantification of CD8+ T lymphocytes producing tumor necrosis factor alpha spots in response to peptide antigens." JOURNAL OF IMMUNOLOGICAL METHODS 1997, vol. 203, no. 2, 1997, pages 141-152, XP002086405 ISSN: 0022-1759 cité dans la demande
- MALARKANNAN SUBRAMANIAM ET AL: "A rare cryptic translation product is presented by K-b major histocompatibility complex class I molecule to alloreactive T cells." JOURNAL OF EXPERIMENTAL MEDICINE 1995, vol. 182, no. 6, 1995, pages 1739-1750, XP000864545 ISSN: 0022-1007
- SHASTRI NILABH ET AL: "Major histocompatibility class I molecules can present cryptic translation Products to T-cells." JOURNAL OF BIOLOGICAL CHEMISTRY 1995, vol. 270, no. 3, 1995, pages 1088-1091, XP000891819 ISSN: 0021-9258
- RONSIN C ET AL: "A non-AUG-defined alternative open reading frame of the intestinal carboxyl esterase mRNA generates an epitope recognized by renal cell carcinoma-reactive tumor-infiltrating lymphocytes in situ." JOURNAL OF IMMUNOLOGY, vol. 163, no. 1, 1 juillet 1999 (1999-07-01), pages 483-490, XP002135539

## Description

La présente invention concerne un composé peptidique, entraînant une réponse T spécifique des tumeurs, qui comprend une séquence d'au moins 8 acides aminés consécutifs de la séquence peptidique codée par une séquence en phase décalée du gène iCE. L'invention porte également sur une composition pharmaceutique comportant ledit composé peptidique et sur l'utilisation de ces composés pour la fabrication d'un médicament destiné au traitement du cancer, en particulier au traitement des tumeurs solides.

Divers produits s'avèrent être reconnus par des cellules T réactives vis-à-vis des tumeurs, la plupart d'entre eux étant isolés à partir de patients présentant des mélanomes. Certains de ces antigènes (Ag) représentent des produits de gènes non mutés dont l'expression dans des tissus d'adultes normaux est limitée aux testicules (MAGE-1, MAGE-3, BAGE et GAGE) (1-4). D'autres gènes non mutés sont des antigènes de différenciation qui sont aussi exprimés, par exemple, dans des mélanocytes normaux mais pas dans d'autres tissus normaux. Ceux-ci comprennent les produits géniques de lignée mélanocytaire MART-1/MelanA (5, 6), gp100 (6), tyrosinase (7, 8) et gp75 (9). Les cellules T réactives vis-à-vis des mélanomes s'avèrent aussi reconnaître des produits mutés des gènes de α-caténine (10), MUM 1 (11) et CDK-4 (12). Les cellules T réactives vis-à-vis du carcinome de cellules rénales (RCC) s'avèrent aussi reconnaître des produits de gènes mutés ponctuellement tels que HLA-A2 (13) ou HSP70-2 (14).

En outre, certains Ag reconnus par des cellules T réactives peuvent être générés par des produits de transcription modifiés, comprenant des séquences introniques, comme dans le cas de MUM-1 (11), N-acétylglucosaminyltransférase-V (GnT-V) (15) ou gp100 (16). La surveillance par les cellules T de l'intégrité des cellules peut se focaliser sur des peptides codés par une phase de lecture ouverte (ORF) alternative située à l'intérieur de l'ORF primaire, comme dans le cas de gp75/TRP-1 (17) et NY-E50-1 (18). Peu d'exemples existent dans la littérature sur l'utilisation des ORF alternatives dans les eucaryotes et la signification biologique des produits correspondants est inconnue. Néanmoins, on peut supposer que ces produits pourraient servir de cibles antigéniques et augmenter l'efficacité de la surveillance immunitaire. En effet, Il y a une relation de plus en plus nette entre le contrôle traductionnel anormal de l'expression de gènes (comme pour c-mys ou FGF-2) (19-21) et l'apparition d'un cancer, et donc les peptides immunogéniques dans des tumeurs peuvent provenir de peptides dérivant de l'ORF primaire mais également d'ORF alternatives.

Le criblage d'une bibliothèque d'ADNc avec un clone de cellules T réactives vis-à-vis du carcinome de cellules rénales (RCC) de restriction HLA-B7, dérivant de lymphocytes infiltrant les tumeurs (TIL) qui ont été amplifiés par clonage in vivo, a conduit dans le cadre de la présente invention à l'isolation d'un nonamère codé par une phase ouverte de lecture (ORF) alternative (A + 1 décalage de phase) du gène de la carboxyle estérase intestinale (iCE). Ce peptide se fixe aux molécules présentant HLA-B*0702, comme déterminé dans un test de fixation par immunofluorescence en utilisant des cellules T2 transfectées. L'expression constitutive de cette protéine d'ORF alternative a été observée dans toutes les lignées de cellules rénales HLA-B7* transformées qui étaient reconnues dans des essais de cytotoxicité par les TIL. Le gène de iCE est transcrit dans des tumeurs RCC ainsi que dans des tissus normaux de foie, d'intestin ou de rein. Une mutation du site d'initiation de traduction ATG naturel n'altère pas la reconnaissance, ce qui montre que le décalage de cadre (c'est-à-dire le glissement du ribosome vers l'avant) et le recodage ne sont pas les mécanismes impliqués. De plus, une mutation ponctuelle des trois codons AUG pouvant servir de sites d'initiation de traduction alternatifs dans l'ORF +1 n'abolit pas la reconnaissance, tandis que la mutation d'un codon ACG en amont le fait, indiquant que ce dernier codon initie la traduction de l'ORF alternative. De manière inattendue, cette ORF alternative est donc initiée à partir d'un codon cryptique non-AUG (ACG).

### Description

L'invention concerne un composé peptidique, entraînant une réponse T spécifique, caractérisé en ce qu'il comprend la séquence SEQ ID N°1 suivante : ou une partie de la SEQ ID No 1 comprenant au moins la séquence SPRWWPTCL (SEQ ID N°2).

L'invention a pour objet un composé peptidique de la séquence SEQ ID N°1 présentant au moins une mutation ou une altération par rapport à la séquence SEQ ID N°1, et ce qu'il entraîne une réponse T spécifique. Un tel composé peptidique peut notamment dériver de la séquence SPRWWPTCL (SEQ ID N°2).

On entend par « composé peptidique » dans le cadre de l'invention, une entité constitué au minimum par un fragment peptidique dérivé du polypeptide codé par une ORF alternative A+1 ou A+2 de iCE ou par une succession desdits fragments peptidiques, et ayant éventuellement un ou plusieurs autres éléments différents des acides aminés naturels ou non naturels. Ces éléments ont pour but de protéger chimiquement ou physiquement lesdits fragments peptidiques, et/ou de favoriser leur absorption par l'organisme, et/ou leur administration, et/ou leur biodisponibilité. Par exemple, cette protection permet aux peptides d'atteindre leurs cibles sans subir l'action de diverses protéases présentes dans l'organisme. De telles modifications chimiques peuvent également augmenter l'affinité d'un peptide antigénique pour les molécules HLA-A2 et permettre une efficacité accrue du vaccin in vivo, Rosenberg et al, (1998).
Lesdits éléments peuvent être par exemple:
- Des groupes chimiques protecteurs connus par l'homme du métier réagissant aux extrémités NH2 et/ou COOH d'un peptide, cette modification ne baissant pas significativement le caractère immunogénique du peptide.
- Des groupes chimiques améliorant l'efficacité du vaccin in vivo.
- Des lipides ou des acides gras que l'on attache de façon covalente aux fragments peptidiques pour former des composés peptidiques appelés lipopeptides. L'acide palmitoïque en est un exemple parmi d'autres, Vitiello et al, (1995), incorporé dans la description par référence.
- Une protéine porteuse desdits fragments peptidiques possédant des sites de restriction et permettant l'acheminement des fragments peptidiques intactes jusqu'à leurs sites d'action dans l'organisme.

Ainsi, le composé peptidique selon l'invention peut comporter au moins un élément différent des acides aminés naturels.

Un mode supplémentaire de réalisation de l'invention concerne un fragment d'ADN codant au moins pour un fragment peptidique défini ci-dessus. Ce fragment peut comprendre la séquence SEQ ID N°3 suivante :

Cette séquence correspond à l'ORF alternative A+1, du gène de iCE qui est exprimée dans les cellules tumorales. Le produit d'expression de cette ORF est reconnu par un clone de cellules T réactives vis-à-vis du RCC à restriction par HLA-B7. Les TIL réactifs sont amplifiés in situ sur le site de la tumeur.

On entend par « fragments d'ADN » des fragments simples brins, double brins, d'ADN, d'ADNc et/ou d'ARN. La séquence nucléotidique correspondant à la séquence d'acides aminés desdits fragments peptidiques peut varier de manière à comprendre tous les différents codons possibles pour un acide aminé donné selon le principe de la dégénérescence du code génétique. La présente invention a également pour objet un vecteur d'expression d'un fragment peptidique contenant un fragment d'ADN ci-dessus mentionné, fusionné à un promoteur fort et efficace dans les cellules eucaryotes, et/ou dans les cellules procaryotes, en particulier dans les cellules humaines. Le vecteur peut être viral, plasmidique, ou un pseudo-vecteur et peut comporter des marqueurs de sélection et exprimer des adjuvants immunologiques tels que des cytokines et/ou des lymphokines.
L'invention concerne également des cellules dendritiques chargées en composés peptidiques, et des cellules dendritiques transformées par le vecteur d'expression exprimant les fragments peptidiques. Ces cellules peuvent être aussi des macrophages. Nestle et al, (1998), décrit une méthode de vaccination qui consiste à charger les cellules dendritiques prises à un patient par des peptides antigéniques (en culture in vitro), et les injecter dans le système lymphatique de ce même patient.

Un autre aspect de l'invention a pour objet une composition pharmaceutique comprenant un composé peptidique ou un mélange de composés peptidiques selon l'invention et un véhicule pharmaceutiquement acceptable. Cette composition peut comporter en outre un ou plusieurs adjuvants immunologiques, notamment des facteurs cytotoxiques des tumeurs.
L'invention porte aussi sur une composition pharmaceutique comprenant un vecteur d'expression tel qui mentionné ci-dessus et un véhicule pharmaceutiquement acceptable ou un fragment d'ADN selon l'invention ou bien encore les cellules indiquées ci-dessus et un véhicule pharmaceutiquement acceptable.

La composition pharmaceutique ou le produit de combinaison selon l'invention peut comporter en outre un ou plusieurs adjuvants immunologiques, notamment des agents cytotoxiques des tumeurs. Ces produits peuvent comporter un véhicule pharmaceutique compatible avec une administration IV, subcutanée, orale, ou nasale, de préférence sélectionné parmi les liposomes, les nano-particules, ou les émulsions lipidiques, chargés positivement ou négativement.

Un autre aspect de l'invention porte sur l'utilisation d'un composé peptidique tel que défini ci-dessus pour la fabrication d'un médicament, notamment destiné au traitement du cancer, en particulier des tumeurs solides, notamment des carcinomes, des mélanomes, des neuroblastomes, de préférence des hépatocarcinomes et des adénocarcinomes du côlon ou du rein. Ce médicament peut être destiné à une immunisation ex vivo, consistant notamment à induire in vitro des CTL spécifiques de tumeurs, les expandre et les réinjectés, ladite induction pouvant être effectuée à l'aide de cellules dendritiques chargées ou à une immunisation in vivo. L'invention porte aussi sur l'utilisation dudit composé peptidique pour augmenter en milieu de culture la population des CTL des tumeurs et/ou induire la sécrétion par lesdits CTL de facteurs cytotoxiques tels que par exemple IL-2, IFN-γ et TNF et/ou pour stimuler les défenses immunitaires, en particulier pour augmenter la population des CTL des tumeurs et/ou induire la sécrétion par lesdits CTL de facteurs cytotoxiques tels que par exemple IL-2, IFN-γ et TNF.

L'invention vise également un anticorps monoclonal se liant au composé peptidique ayant la séquence SEQ ID No 1 dans un essai immunologique .

L'invention vise également un procédé de détection d'un peptide ou polypeptide codé par l'ORF A+1 de iCE, comprenant les étapes consistant à :
a) Mettre en contact un échantillon prélevé chez un individu avec un anticorps monoclonal mentionné ci-dessus,
b) permettre la formation du complexe anticorps-peptide ou polypeptide,
c) détecter ledit peptide ou polypeptide au moyen d'un marqueur détectable dans le complexe ou qui se lie au complexe ;
et une trousse de diagnostic comportant notamment ledit anticorps pour la détection du cancer, notamment pour le prognostique du cancer déclaré chez un individu.
Une composition comprenant notamment ledit anticorps monoclonal et un véhicule pharmaceutiquement acceptable peut également être utile dans le cadre du traitement du cancer.

L'ADNc de iCE a été isolé à l'origine à partir d'une bibliothèque d'ADNc d'intestin grêle humain (31). Il présente une homologie de 65 % avec d'autres carboxyle estérases de différentes espèces mammifères. Il est exprimé dans l'intestin, le foie ou le rein humain et semble jouer un rôle important pour le contrôle xénobiotique et la détoxication de la muqueuse intestinale (31). Une grande série d'épitopes de cellules T codés dans la région nucléotidique minimale de la ORF de iCE régulière a été testée et aucun d'entre eux n'a été reconnu dans le contexte de l'élément de restriction HLA-B*0702 de classe I. Au contraire, une ORF de 453 nt codée dans cette région suivant un décalage de phase +1 s'est avérée coder pour un nonamère avec des résidus d'ancrage à HLA-B7 sur les positions 2, 3 et 9 (SPRWWPTCL, SEQ ID n°2). Une lyse semi-maximale a été obtenue avec moins de 10⁻⁶ M de nonapeptide dans des essais de sensibilisation de cible (target sensitization assays). La fixation de ce nonapeptide à des cellules T2 transfectées par HLA-B*0702 est stable dans le temps, suggérant que de faibles quantités de l'expression de cette ORF alternative sont suffisantes pour induire une reconnaissance de cellules T in vitro ainsi qu'une prolifération de cellules T in vivo, comme montré, pour ce dernier cas, par l'amplification in situ au niveau du site tumoral de la sous-population de TIL correspondante.

Les résultats obtenus dans le cadre de l'invention révèlent qu'un nouveau mécanisme est impliqué dans la génération d'épitopes de cellules T. Une ORF alternative induite par un codon non-AUG cryptique conduisant à une phase de lecture traductionnelle +1 s'est avérée coder un Ag de tumeur reconnu par des TIL. Dans deux autres exemples, gp75/TRP-1 (17) et NY-E50-1 (18), des peptides reconnus par des TIL sont codés par une ORF alternative située à l'intérieur de la ORF primaire. Un mécanisme par lequel l'ORF alternative est traduite a été suggéré pour gp75/TRP-1 (17), où la reconnaissance était affectée par la présence d'un AUG interne précédant l'épitope. En plus de ce mécanisme de criblage ribosomal, un décalage de phase ribosomal (39, 40) a été suggéré pour la production d'épitopes de cellules T (41) mais, dans le cas du gène iCE, cette possibilité est exclue car la mutation du site d'initiation de traduction ATG naturel n'affecte pas la reconnaissance des peptides. En fait, la présence du premier site d'initiation de traduction interne cryptique (un codon ACG en position 440) dans la ORF alternative +1 de iCE est suffisante pour diriger l'expression de quantités suffisantes de peptide de iCE pour l'activation de cellules T in vitro ainsi que in vivo (c'est-à-dire conduisant à une expansion clonale de cellules T in situ). Le modèle de balayage fuyant où des ribosomes évitent occasionnellement le premier AUG avec une médiocre séquence consensus de Kozak et initient une traduction sur un AUG en aval peut s'appliquer à iCE du fait de la présence d'une pyrimidine en position +4 à la place d'une purine.

A notre connaissance, ceci est le premier exemple d'un épitope codé par une ORF alternative non définie par ATG et reconnu par des cellules T à réactivité tissulaire dans une maladie humaine. Des lignées de cellules rénales HLA-B7+ non transformées, établies in vitro, ont été reconnues dans des essais de cytotoxicité par le clone 3B8 dérivé de TIL. Il a été montré que des initiations de traduction alternatives, non définies par ATG, de la molécule du facteur 2 de croissance de fibroblastes, sont induites dans des cellules stressées ou transformées, par comparaison à celles définies par ATG (20). De façon similaire, l'expression de formes de iCE non initiées par ATG peuvent être régulées positivement dans les tumeurs, conduisant à l'expansion clonale in situ des TIL correspondants. Cette ORF alternative de iCE exprime donc un nouvelle Ag de tumeur intéressant à utiliser en immunothérapie notamment chez des patients présentant un hépatocarcinome ou un adénocarcinome du côlon ou rénal. Plus généralement, les résultats obtenus montrent la possibilité que des ORF alternatives induites par des codons non-AUG dans les 3 phases de lecture traductionnelles puissent coder des épitopes de cellules T dans certaines maladies humaines, telles que le cancer ou des affections auto-immunes.

Pour la suite de la description, on se référera aux légendes des figures présentées ci-dessous.

### Légendes

### Figure 1 :

### (A) Lyse spécifique de lignée cellulaire RCC-1 autologue par le clone 3B8 de CTL.

La cytotoxicité du clone 3B8 vis-à-vis de la lignée cellulaire RCC-1 autologue et des cellules K562 a été testé dans un essai de relargage du chrome standard à différents rapports E:T. Le blocage de la lyse par le mAb W6.32 est également représenté.

### (B) Cytotoxicité de 3B8 vis-à-vis de diverses lignées de cellules allogéniques.

3B8 a été testé avec la lignée RCC-1 autologue et diverses lignées de cellules RCC allogéniques (RCC-3, RCC-4, RCC-5) dans un essai au chrome standard à un rapport E:T de 18:1. A titre de témoin, le mAb W6.32 a été utilisé pour bloquer les molécules HLA de classe I impliquées dans la présentation d'antigène.

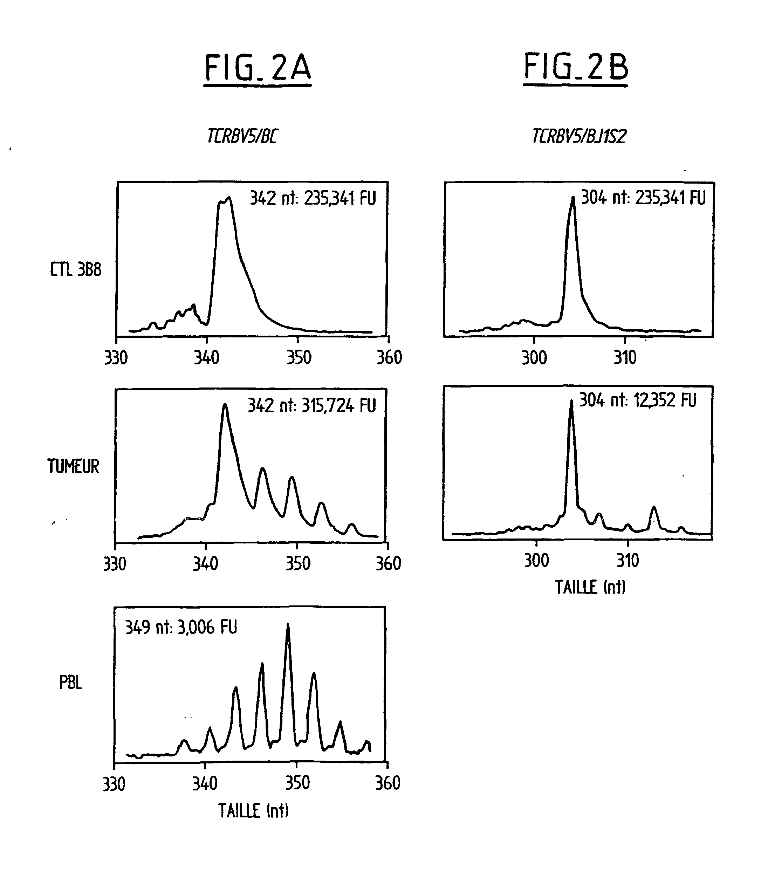

### Figure 2 : Analyse de taille de CDR3 dans des TIL et dans le clone 3B8 utilisant des amorces sélectionnées TCRBV (A) et TCRBJ (B).

L'ARN a été soumis à une transcription inverse et à une amplification sur 40 cycles en utilisant les amorces TCRBV5 et BC. L'ADN obtenu a été copié sur 5 cycles dans une réaction d'élongation utilisant l'amorce fluorescente nidifiée TCRBC (A) ou TCRBJ152 (B) (13 amorces BJ testées, BJ1S1-BJ1S7, BJ2S1-BJ2S6). Les produits amplifiés ont été analysés sur un séquenceur automatisé. Les profils obtenus montrent les tailles en nt (axe des x) et l'intensité de fluorescence (axe des y) des produits amplifiés. Les valeurs FU absolues obtenues pour les pics dominants sont indiquées.

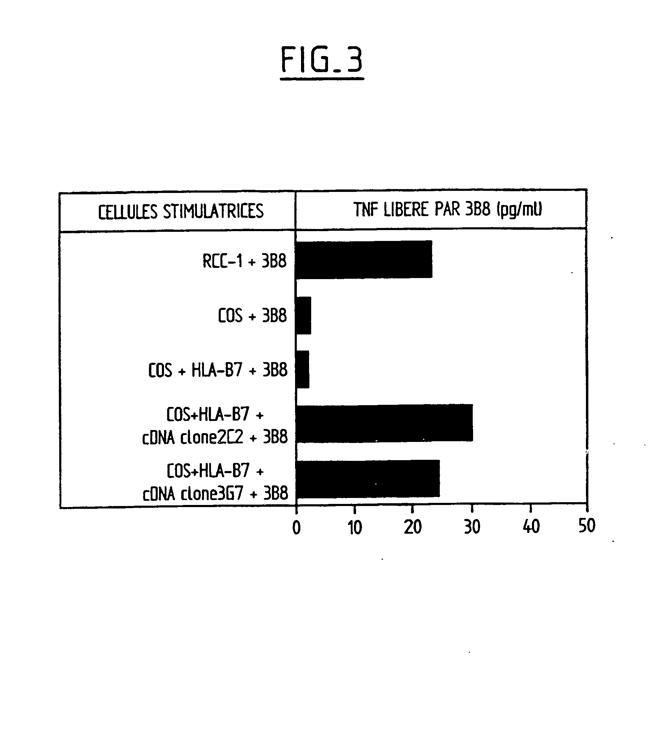

### Figure 3 : Stimulation de 3B8 de CTL par des cellules COS-7 co-transfectées de façon transitoire avec le vecteur d'expression pcDNAI contenant le clone d'ADNc 2C2 ou 3G7 et l'ADNc de HLA-B*0702 autologue.

Les cellules stimulantes témoins comprennent la lignée cellulaire RCC-1 servant de témoin positif et des cellules COS-7 transfectées avec de l'ADNc de HLA-B*0702 seul servant de témoin négatif. L'ADNc de iCE a été co-transfecté de façon transitoire dans des cellules COS-7 avec l'ADNc de HLA-B*0702 et le clone 3B8 a été ajouté au bout de 48 heures. La production de TNF a été déterminé par son effet cytotoxique sur des cellules WEHI 18 heures plus tard. Les cellules stimulantes témoins comprennent la lignée cellulaire RCC-1 servant de témoin positif et des cellules COS-7 transfectées avec HLA-B*0702 seul servant de témoin négatif.

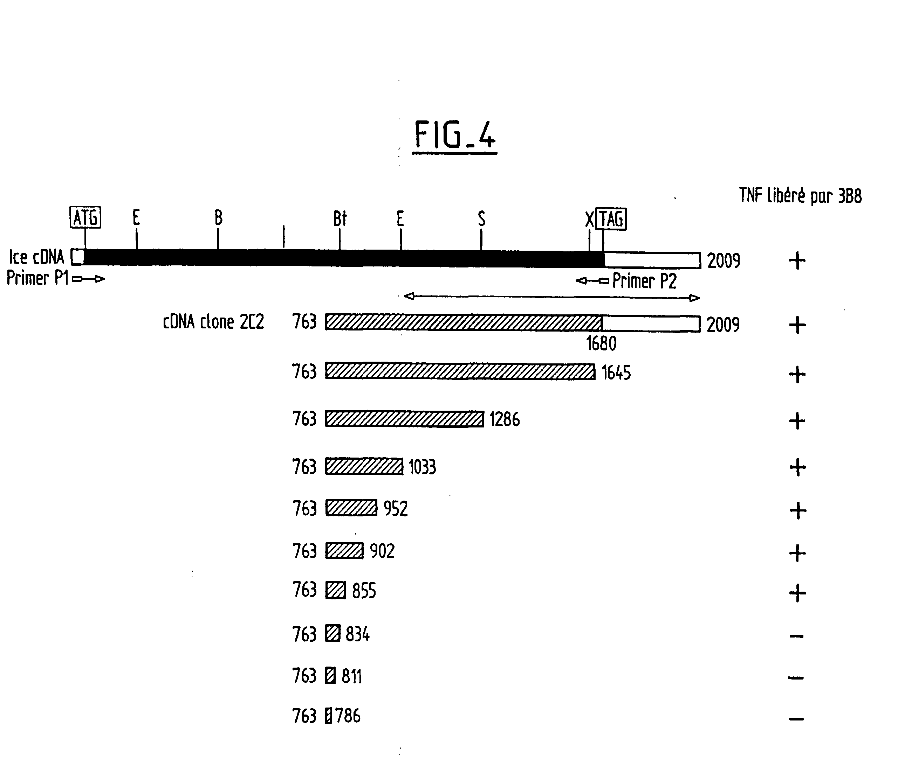

### Figure 4 : Localisation de la séquence d'ADNc de iCE codant pour le peptide antigénique reconnu par 3B8.

Il s'agit d'une représentation schématique de la séquence d'ADNc de iCE de pleine longueur, du clone 2C2 d'ADNc et de divers 2C2 d'ADNc tronqués. Les régions 3' et 5' non traduites sont représentées par des cadres soulignés. La séquence traduite de iCE humaine est représentée par un cadre plein ; le clone 2C2 d'ADNc est représenté par un cadre pointillé et les 2C2 d'ADNc tronqués sont indiqués par des cadres hachurés. Les nucléotides sont numérotés à partir du codon non-sens ATG naturel. Les petits cadres noirs avec une flèche indiquent l'emplacement de l'amorce P1 et de l'amorce P2. L'ADNc utilisé comme sonde pour l'hybridation du transfert d'ARN est indiqué par une flèche à deux pointes. Sites de restriction (B : Bam HI ; Bs : BstX I ; E : EcoR I ; S : Sma I ; X : Xba I). La reconnaissance par 3B8 de CTL des cellules COS-7 transfectées de façon transitoire avec l'ADNc de HLA-B*0702 autologue et divers ADNc tronqués est indiquée. Les cellules transfectées ont été incubées pendant 24 heures avec 5000 cellules 3B8 et la quantité de TNF a été mesurée dans les surnageants par l'effet de cytotoxicité sur des cellules WEHI-13.

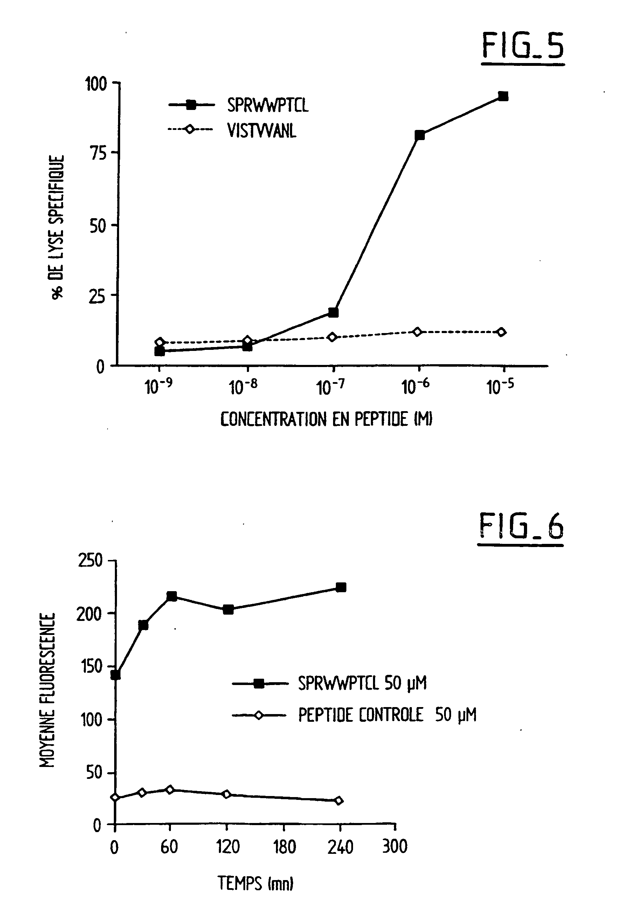

### Figure 5 : Lyse de lignées de cellules transformées par EBV autologues incubées avec le peptide codé de iCE par 3B8 de CTL.

2000 cellules transformées par EBV ont été incubées et marquées au 51 Cr pendant 1 h en présence du peptide de iCE à restriction HLA-B7 ou un autre peptide témoin à restriction HLA-B7. Le clone 3B8 a ensuite été ajouté à titre d'effecteur en un rapport fixé à 30:1. On a mesuré le relargage du chrome au bout de 4 h.

### Figure 6 : Induction de l'expression de HLA-B7 sur des cellules T2 par le peptide de iCE.

Des cellules T2 ont été incubées à 26°C pendant 16 heures dans du milieu sans sérum avec des peptides à une concentration de 50 µM. Puis les peptides ont de nouveau été ajoutés et les cellules ont été incubées à 37°C. A intervalles de 30 min ou 1 h, des aliquotes de cellules ont été recueillies et on a contrôlé le changement d'expression de HLA-B7 par cytométrie de flux avec un mAb and-HLA-B7 (HB59). A titre de témoin, un peptide HSP70 à restriction HLA-A2 a été utilisé.

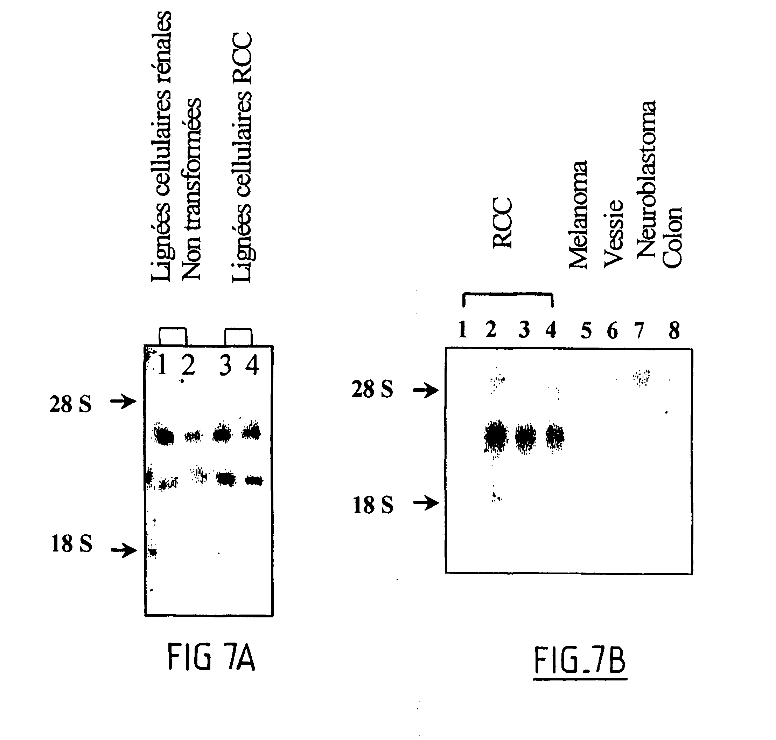

### Figure 7 : Analyse de produits de transcription d'ARN de iCE dans diverses lignées cellulaires (A) et divers fragments de tumeur (B).

5µg d'ARN poly(A)+ (A) et dix µg d'ARN total (B) ont été chargés sur un gel dénaturant de formaldéhyde à 1 % d'agarose. L'ARN a été transféré sur une membrane et le transfert d'ARN a été hybridé avec un fragment de clone 2C2 d'ADNc marqué au 32P, servant de sonde. Une hybridation a été effectuée avec une sonde de glycéraldéhyde-3-phosphate déshydrogénase (GAPDH) à titre de témoin interne pour la charge de quantités uniformes d'ARN pour l'analyse (non représenté).

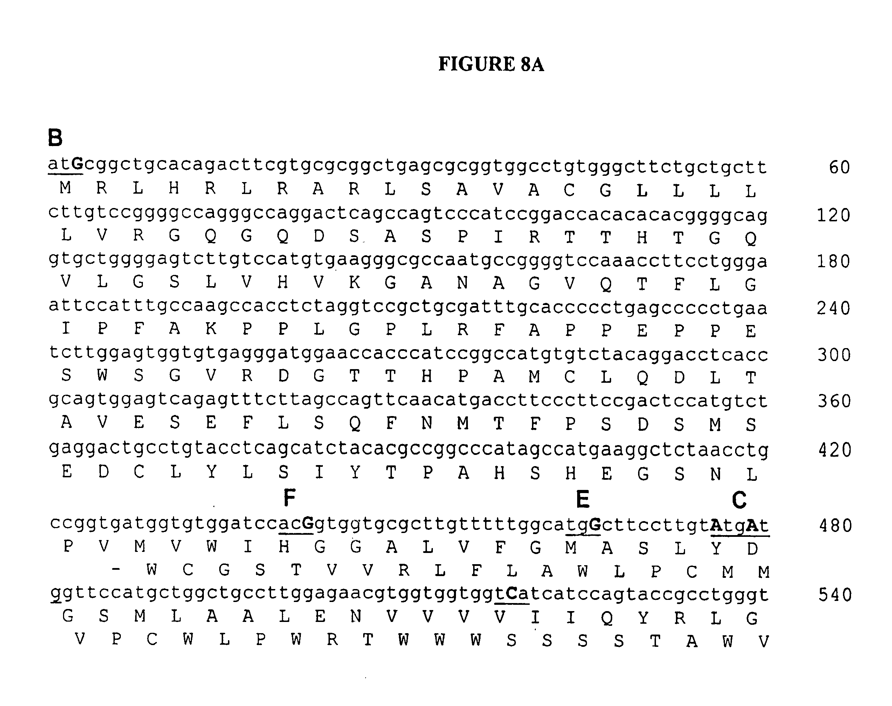

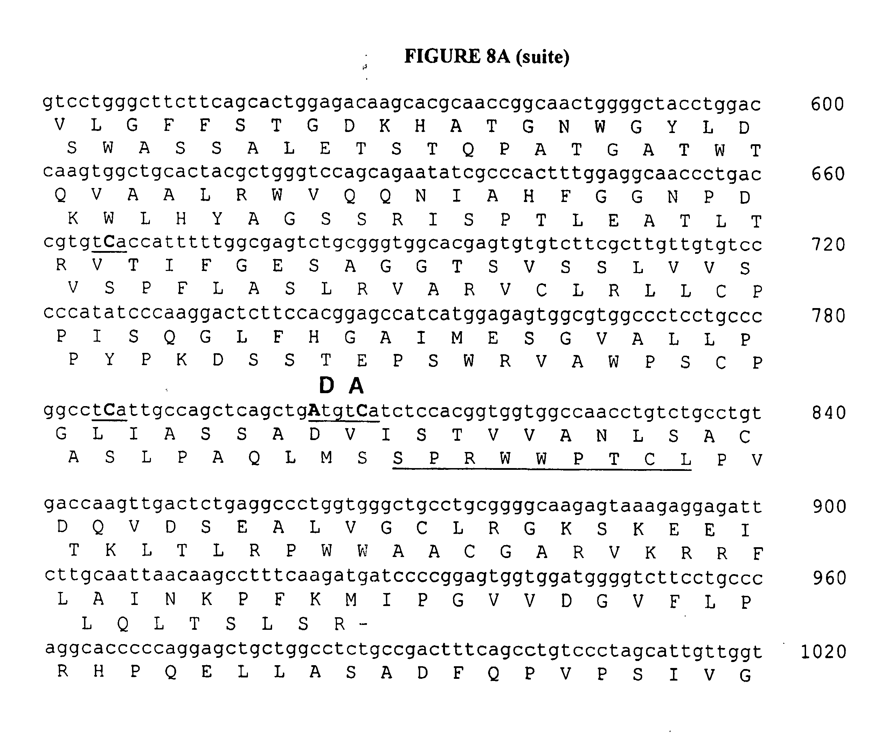

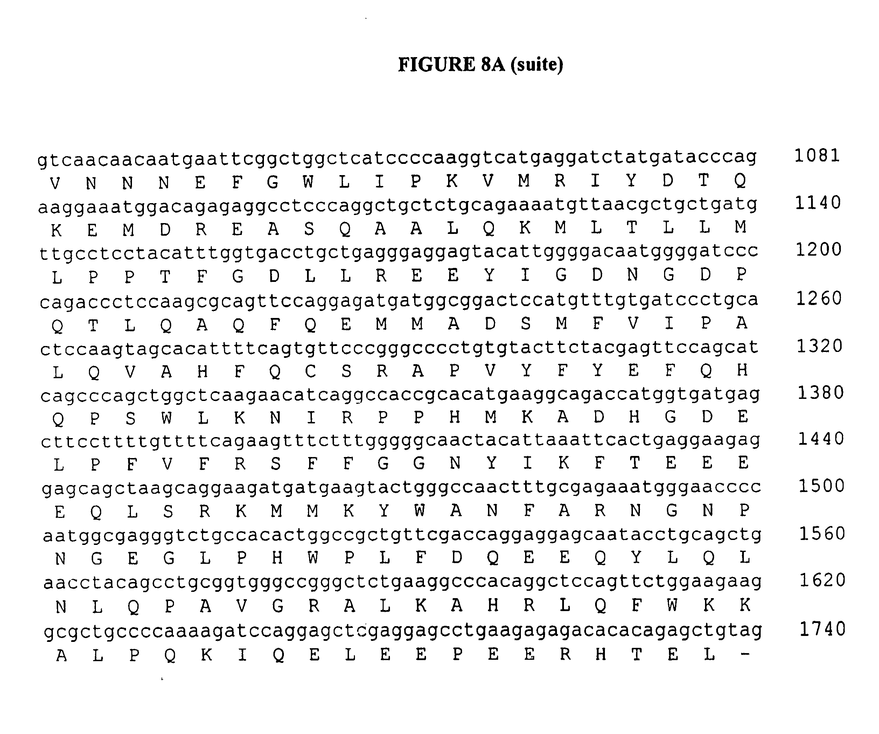

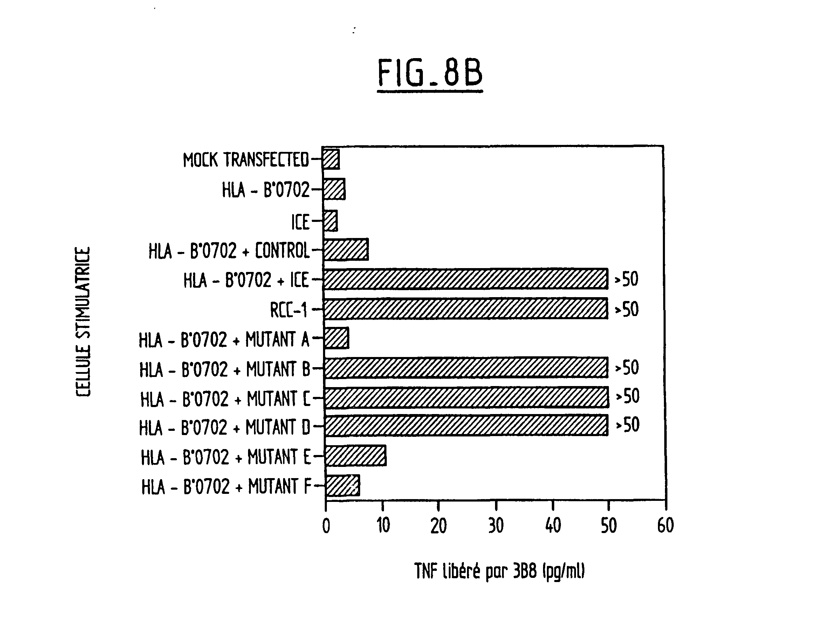

### Figure 8 : Une phase ouverte de lecture non définie par ATG de iCE est reconnue par le 3B8 de CTL.

(**A**) Séquence de la région codante d'ADNc de iCE avec les phases ouvertes de lecture primaire et alternative (décalage a+1). Les positions des nucléotides (nt) mutés sont représentées en lettres capitales grasses, les codons correspondants sont soulignés et l'emplacement des mutants A-F testés (B) est indiqué au-dessus des codons mutants. La séquence du peptide antigénique codé par la ORF +1 est soulignée.
(**B**) L'aptitude de mutants ponctuels (A-F) à stimuler la libération de TNF à partir du clone 3B8 après co-transfection avec HLA-B*0702 dans des cellules COS a été testée. Les témoins négatifs comprennent une transfection simulée avec HLA-B*0702 ou l'ADNc de iCE seul ou une co-transfection avec HLA-B*0702 et un plasmide de pcDNAI témoin.

### Exemple 1 : Matériels et méthodes

### Lignées cellulaires

Des cellules K562 ont été cultivées et la lignée de cellules B transformées par EBV provenant du patient 1 dans du milieu constitué de RPMI (Gibco-BRL, Paisley, GB) supplémenté avec 1 % de L-glutamine 200 mM, 1 % de pyruvate de sodium 200 mM, 1 % de Hepes, 5 % de sérum de veau foetal (FCS) et 50 UI/ml de pénicilline (Gibco-BRL, Paisley, GB) a été obtenue. Le clone 13 de WEHI-164 (W13) et les cellules COS-7 ont été cultivées dans du RPMI (Seromed, Biochrom KG, Berlin) supplémenté avec 1 % de L-glutamine 200 mM, 1 % de pyruvate de sodium 200 mM, 1 % de Hepes, 5 % de sérum de veau foetal (FCS) et 50 UI/ml de pénicilline.

### Patients et établissement des lignées de cellules RCC

Les lignées de cellules RCC ont été établies comme décrit antérieurement (22). Des tumeurs primaires ont été obtenues auprès de patients non traités qui avaient subi une néphrectomie radicale. La lignée de cellules RCC-1 a été établie à partir du patient 1 (HLA A1, A32, B7, B12-44, Cw5, Cw7), un homme âgé de 56 ans ayant un carcinome de cellules rénales clair et granulaire sans métastases. Après chirurgie, on a traité des fragments par digestion enzymatique et on a cultivées les suspensions de cellules tumorales dans du milieu RCC complet (22). Les lignées de cellules tumorales RCC-2 (HLA A1, A3, B7, B8, Cw7, Cw7), RCC-3 (HLA A1, A29, B22, B15-62/63, Cw1, Cw7-17), RCC-4 (HLA A3, A19-29, B7, B12-44, Cw7, Cw16), RCC-5 (HLA A1, A3, B6, B22-56, Cw1, Cw7), RCC-6 (HLA A9-24, A32, B12-44, B18, Cw5, Cw5), RCC-7 (HLA A1, A28-68, B8, B40-60, Cw3, Cw7), RCC-8 (HLA A2, A10-25, B18, B13, Cw8, Cw6) dérivant de la tumeur primaire des patients 2, 3, 4, 5, 6, 7 et 8, respectivement, ont été maintenues dans du milieu RCC complet.

### Génération de CTL à partir de TIL du patient 1

Des TIL autologues ont été générés à partir d'une suspension décongelée de cellules tumorales dissociées. Une culture mixte autologue de lymphocytes/cellules tumorales (MLTC) a été réalisée comme suit : Au jour 1, des cellules tumorales dissociées ont été ensemencées à raison de 2 x 10⁶ TIL dans des plaques à fond plat à 6 puits (Falcon, Becton Dickinson, New Jersey) dans du RPMI 1640 (Gibco-BRL, Paisley, GB) contenant 1 % de L-glutamine 200 mM, 1 % de pyruvate de sodium 200 mM, 8 % de sérum AB humain (Institut Jacques Boy, S.A., Reims, France), 50 UI/ml de pénicilline, supplémenté avec 5 % de facteur de croissance de cellules T (TCGF) et 50 UI/ml d'interleukine-2 humaine (rIL-2) (Roussel Uclaf, Romainville, France), appelé ci-après "milieu complet MLTC". Le milieu complet MLTC a été retiré tous les trois jours selon les besoins, et on l'a remplacé par du milieu MLTC complet frais. Aux jours 7, 15, 21, 2 x 10⁶ TIL ont été re-stimulées avec 2 x 10⁵ cellules tumorales autologues irradiées (100 Gray) ensemencées dans des plaques à fond plat à 6 puits avec du milieu complet MLTC. Au jour 15, l'activité cytotoxique des TIL a été testées contre les lignées cellulaires RCC-1 et K562 autologues, le phénotype de surface par immunofluorescence directe a été caractérisé et les cellules ont été clonées par la technique des dilutions limites. Les TIL ont été ensemencés à raison de 0,6 à 600 cellules/puits dans des plaques à 96 micropuits en forme de V (Nunc, Danemark), ensemencées au préalable avec des cellules tumorales autologues irradiées (1 x 10⁴/puits) servant de stimulateurs et des PBL allogéniques irradiés (8 x 10⁴/puits) et des cellules B transformées par EBV irradiées (2 x 10⁴/puits), servant de cellules nourricières, dans un volume total de 200 µl de milieu complet MLTC. Tous les 3 jours, 60 µl de surnageant a été rétiré dans chaque puits, et remplacé par 60 µl de milieu frais. La cytotoxicité des clones a été déterminée dans un essai de relargage du chrome standard de 4 h. Tous les 7-10 jours, des clones de CTL ont été re-stimulés avec la lignée de cellules nourricières allogéniques et la lignée de cellules tumorales autologues, comme décrit ci-dessus.

### Essai de cytotoxicité

L'activité cytolytique des CTL a été déterminée dans un essai de relargage du ⁵¹Cr standard comme décrit antérieurement (22). Des cellules cibles ont été marquées (lignées de cellules RCC, K562) pendant 1 h avec 50 µCi à 100 µCi de ⁵¹Cr (Du Pont, NEN, Boston, MA) à 37°C, et 2 x 10³ cellules ont été ensemencées dans des plaques à 96 micropuits dans 100 µl de RPMI supplémenté avec 5 % de FCS. Des cellules effectrices ont été rajoutées dans les puits, à différents rapports E:T allant de 40:1 à 0,1:1. Pour l'inhibition de la lyse par des mAb, des cellules cibles ont été pré-incubées pendant 2 h en présence d'une concentration saturante de mAb avant addition des cellules effectrices. Les plaques à 96 micropuits ont été incubées à 37°C pendant 4 h et la libération de ⁵¹Cr a été déterminée dans les surnageants recueillis. Pour le blocage de la cytotoxicité ou la production de TNF, les mAb suivants ont été utilisées : W6/32, un mAb pan-MHC de classe I, et B1.23.2 (ME1), un mAb spécifique de HLA-B/C.

### Transfection de cellules COS-7 et criblage de produits de transfection

Des expériences de transfection ont été réalisées avec des cellules COS-7 en utilisant la méthode au DEAE-dextrane-chloroquine (5, 7, 23). Trois jours avant la transfection, des cellules COS-7 ont été ensemencées dans des plaques à fond plat à 96 micropuits, à raison de 5 x 10³ cellules/puits, dans 150 µl de RPMI contenant 20 % de FCS. Les expériences de transfection ont été effectuées en double dans deux plaques de micropuits différentes. Pour la transfection, le milieu a été jeté puis remplacé par 30 µl de mélange de transfection contenant 35 µg de DEAE-dextrane (Sigma), 0,1 mM de chloroquine (Sigma), avec 100 ng d'ADN plasmidique représentant un groupe d'environ 200 clones recombinés provenant de la bibliothèque d'ADNc et 100 ng du plasmide HLA-B*0702 autologue. Les cellules COS-7 ont été incubées pendant 4 h à 37°C, puis le milieu a été retiré et les cellules ont été incubées pendant 2 minutes dans un tampon PBS 1x contenant 10 % de solution de diméthylsulfoxyde. Les cellules ont été lavées une fois dans du tampon PBS 1x et on les a incubées avec du RPMI à 10 % de FCS pendant 2 jours. Au bout de 2 jours, l'aptitude des cellules COS-7 transfectées à stimuler la production de TNF par le clone 3B8 a été testée, comme déterminé par l'essai WEHI.

L'aptitude des cellules COS-7 transfectées à stimuler la production de TNF a été testées (24). 2 x 10³ CTL (clone 3B8) ont été rajoutée à des plaques à fond plat à 96 micropuits contenant des cellules COS-7 transfectées de façon transitoire dans 100 µl de RPMI à 10 % de FCS. Après 18 h, chaque surnageant a été recueilli et sa teneur en TNF a été déterminé en testant son effet cytotoxique sur des cellules de clone 13 de WEHI-164 (25) dans un dosage colorimétrique au bromure de 3-[4,5-diméthylthiozol]-2,5-diphényl-tétrazolium (MTT).

### Analyse de taille de CDR3

L'analyse de taille de CDR3 de segments de gène TCRBV exprimés par le clone 3B8 de CTL ou se trouvant dans le sang ou des fragments de tumeur a été effectuée comme décrit précédemment (22). Le mode opératoire utilisé pour l'analyse de taille de CDR3 comprend des amplifications RT-PCR indépendantes de fragments TCRBV-BC (26), suivies d'un écoulement des produits PCR utilisant des amorces nidifiées fluorescentes TCRBC ou TCRBJ (27) et d'une détermination de taille de produits d'écoulement fluorescents par électrophorèse sur un séquenceur d'ADN automatisé ABI 373 (Applied Biosystems, Inc. Foster City, CA) utilisant le logiciel Immunoscope (28). Comme les positions d'amorce 5' et 3' sont fixes, des variations de taille des produits d'écoulement sont dues uniquement à des différences de longueur des régions CDR3. Chaque pic est caractérisé par sa position (taille de CDR3) et une intensité de fluorescence (unités de fluorescence arbitraires ou FU). Les graphiques représentant des motifs de taille de CDR3 ont été étalonnés à 100 % pour les pics les plus hauts. Dans du sang provenant de donneurs sains, la plupart des profils reflétant une diversité de tailles de CDR3 dans une sous-famille Vβ donnée affichaient 5 à 8 pics à intervalles de 3 nucléotides, avec une distribution presque gaussienne (21). Les pics dominants ont été définis comme étant des signaux de forte intensité, avec une diminution considérable dans les autres signaux de CDR3.

### Construction de la bibliothèque d'ADNc

L'ARN poly(A)+ a été extrait à partir de la lignée cellulaire RCC-1 en utilisant une trousse Maxi Message Marker® (R&D systems Abingdon, GB) en suivant les instructions du fabricant. L'ADNc du premier brin a été synthétisé avec le système Superscript Choice System® (Gibco BRL, Gaithersburg, MD) en utilisant une amorce oligo-dT contenant un site Not 1 à son extrémité 5', puis l'ADNc du deuxième brin a été synthétisé. Les ADNc ont été ligaturés en leur extrémité franche avec des adaptateurs semi-Bst XI (InVitrogen), puis digérée avec Not I et fractionnés par chromatographie sur colonnes de Sephacryl S-500 HR. Des fractions d'ADNc ont été sous-clonées dans les sites Bst XI et Not I du vecteur d'expression pcDNAI. Les plasmides recombinés ont été soumis à une électrophorèse dans E. coli MC1061/P3 et les bactéries ont été sélectionnées sur des plaques de gélose LB avec 50 µg/ml d'ampicilline et 10 µg/ml de tétracycline. Dans les expériences de criblage, la bibliothèque d'ADNc de RCC-1 a été divisée en 400 groupes de 200 clones d'ADNc. Chaque groupe de bactéries a été amplifié et l'ADN plasmidique a été extrait en utilisant la méthode de lyse alcaline (29).

### Isolation de l'ADNc de iCE pleine longueur et d'ADNc de iCE mutés par mutation ponctuelle ou tronqués.

L'ARN total a été extrait à partir d'une lignée de cellules RCC par le mode opératoire de centrifugation à l'isothiocyanate de guanidine/chlorure de césium (30). Une transcription inverse a été effectuée sur 5 µg d'ARN total dans un volume réactionnel de 20 µl en utilisant la trousse cDNA Cycle® en suivant les instructions du fabricant. 1 µl du mélange réactionnel d'ADNc a été utilisé dans une réaction PCR utilisant de l'ADN polymérase de Taq (Perkin Elmer). Pour l'amplification d'ADNc de iCE humaine (31), les amorces suivantes ont été utilisées :
- Amorce P1, 5'-CCCAAGCTTGGTGAATAGCAGCGTGTCCGC-3' (nucléotides 28 à 48, sens, SEQ ID N°4).
- Amorce P2, 5'-TGCTCTAGAAGGGAGCTACAGCTCTGTGTG-3' (nucléotides 1666 à 1687, antisens, SEQ ID N°5).

Les conditions pour la PCR sont les suivantes : 10 min à 95°C suivies de 30 cycles d'amplification (94°C pendant 1 min, 60°C pendant 2 min, 72°C pendant 3 min avec une extension finale pendant 10 min à 72°C).

Le produit PCR ainsi obtenu est ensuite digéré par Hind III et Xba I et est sous-cloné dans les sites Hind III et Xba I du vecteur d'expression pcDNAI pour le séquençage. La séquence publiée de l'ADNc de iCE porte le numéro d'accès Y09616. Des mutants de iCE ont été obtenus par mutagenèse dirigée en codant la mutation ponctuelle souhaitée dans des amorces oligonucléotidiques en chevauchement et en générant les mutants par PCR (32). Le séquençage des produits PCR a été réalisé avec une trousse de séquençage d'ADN ABI PRISM (PE Applied Biosystems).

### Analyse par immunotransfert Northern (Northern blot)

L'ARN total a été extrait à partir de diverses tumeurs primaires en utilisant une technique de centrifugation à l'isothiocyanate de guanidinium/chlorure de césium (30). L'ARN poly(A+) a été préparé comme décrit ci-dessus à partir de lignées de cellules RCC et de lignées de cellules rénales non transformées. 5 µg d'ARN poly(A)+ ou 10 µg d'ARN total ont été soumis à une électrophorèse dans un gel de formaldéhyde à 1,2 % d'agarose et on les a transférés sur des membranes en nylon Hybond-N+ (Amersham, GB). L'ARN transféré a été hybridé à la fois avec un fragment d'ADNc 2C2 correspondant aux nucléotides 1033 à 2009 de la séquence d'ADNc de iCE humaine publiée (31) et avec un ADNc de glycéraldéhyde-3-phosphate déshydrogénase (GAPDH) servant de sondes. Toutes les sondes ont été marquées avec de l'alpha[32P]dCTP (3000 Ci mmol-1) en utilisant la trousse de marquage d'amorce aléatoire Prime-IT™ II (Stratagene, La Jolla, CA). L'hybridation a été effectuée à 48°C pendant 16 heures.

Les membranes ont été lavées deux fois avec 2 x SSC à 52°C, une fois pendant 15 minutes avec 0,2 SSC/0,1 % SDS, et ensuite on les a autoradiographiées ou analysées avec un dispositif Phosphor-Imager (Molecular Dynamics, Sunnyvale, Californie, USA).

### Exemple 2 : Un clone de CTL spécifiques de RCC a été isolé à partir des TIL

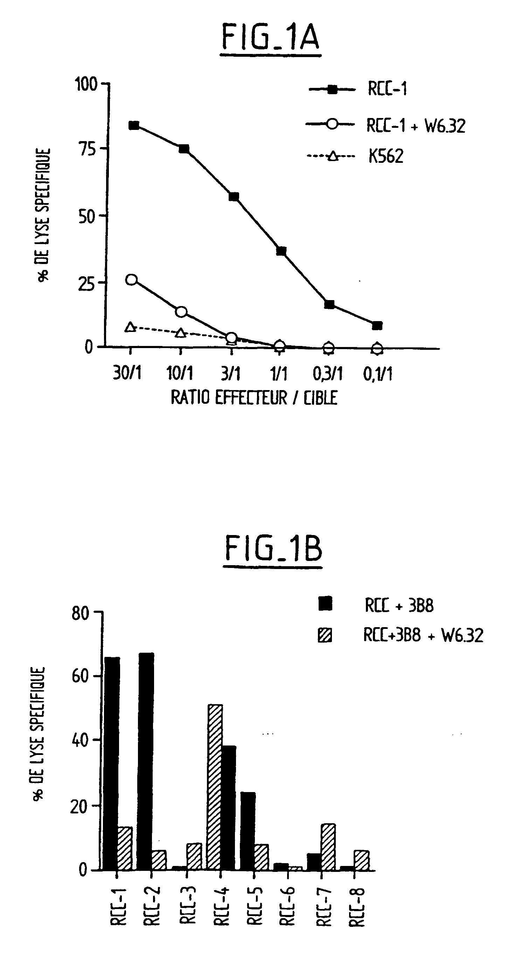

Des TIL provenant du patient 1 ont été stimulées avec des cellules tumorales autologues irradiées en présence d'une faible dose de IL-2 et de TCGF (22). Après 15 jours de MLTC, une activité cytolytique spécifique contre les cellules tumorales autologues (31 % de lyse à un rapport E:T de 40/1) a été détectée et des TIL ont été clonés par la technique des dilutions limites en présence de cellules tumorales autologues, de cellules B transformées par EBV, de PBL allogéniques, avec addition de IL-2 et de TCGF. Un clone TCRα/β⁺ CF8⁺, appelé 3B8, a été isolé. Il lyse la lignée de cellules RCC autologues mais pas les cellules cibles K562 sensibles à la NK. La cytotoxicité du clone 3B8 contre la lignée cellulaire RCC-1 autologue avec du mAb W6/32 a été bloquée (Figure 1A). Dans les essais tant de cytotoxicité (Figure 1B) que de production de TNF, toutes les lignées de cellules RCC HLA-B7⁺ allouéniques (RCC-2, RCC-4, RCC-5 sur la Figure 1B) et aucune des lignées de cellules RCC HLA-B7- (RCC-7, RCC-6, RCC-7, RCC-8) sont reconnues par 3B8. Par conséquent, l'antigène reconnu par 3B8 est présenté par la molécule HLA-B7 et s'avère être couramment exprimé dans les lignées de cellules RCC. Les 6 molécules HLA de classe I ont été isolées à partir de RCC-1 par RT-PCR (33), clonées dans pcDNAI et ont été séquencées. La séquence nueléotidiques de l'ADNc de HLA-B7 autologue a permis d'identifier l'allèle impliqué comme étant HLA-B*0702. Une transfection de cet allèle HLA dans deux lignées de cellules RCC allogéniques HLA-BT s'avère suffisante pour induire une reconnaissance (sécrétion de TNF) par le clone 3B8 de CTL, confirmant le fait que ce clone a conduit à l'identification d'un antigène partagé qui est exprimé par toutes les RCC.

### Exemple 3 : Expansion clonale in situ d'une sous-population de TIL avec des longueurs similaires de CDR3 TCRVB-BC et TCRVB-BJ que le clone 3B8 de CTL spécifiques de RCC

Pour le clone 3B8, un signal a été obtenu avec une seule des 24 amorces de sous-famille Vβ (TCRVB5) et une seule des 13 amorces TCRBJ (TCRBJ1S2) testées. L'analyse de distribution de taille de CDR3 a montré que le clonotype TCRBV5J1S2 de 3B8 est dominant dans la tumeur (comme indiqué par les amorces TCRBV5-BC sur la Figure 2A et par les amorces TCRBV5-BJ12 pour une analyse plus affinée sur la Figure 2B), tandis qu'un tel clonotype n'était pas trouvé dans les PBMC (une distribution de longueur de CDR3 pratiquement gaussienne avec les amorces TCRBV-BC, voir Figure 2A). Ce résultat suggère fortement que le clone 3B8 est entré en expansion spécifiquement sur le site de la tumeur, comme montré antérieurement par un séquençage d'ADNc dans plusieurs cas (14,34-36).

### Exemple 4 : Identification d'un ADNc codant pour l'antigène

Une bibliothèque d'ADNc a été construite dans le vecteur d'expression pcDNAI provenant d'ARN extrait à partir de la lignée cellulaire RCC-1. Cette bibliothèque d'ADNc a été divisée en 400 groupes de 200 plasmides recombinés, et chaque groupe a été co-transfecté en double dans des cellules COS-7 avec le vecteur d'expression pcDNAI contenant l'ADNc codant pour le HLA-B*0702 autologue. L'aptitude des cellules COS-7 à stimuler la production de TNF par 3B8 a été testée. Au bout de 48 heures, les cellules COS-7 co-transfectées ont été incubées pendant 24 heures avec 3B8 et mesuré la concentration de TNF a été mesurée dans les sumageants de culture par son effet cytotoxique sur des cellules WEHI. Les quantités de TNF trouvées dans les surnageants vont de 8 à 11 pg/ml sauf pour deux paires de doublons plus importants (14 et 15 pg/ml). Pour chaque groupe de bactéries correspondant à ces puits candidats, l'ADN plasmidique a été extrait et sous-cloné. Un deuxième criblage en transfectant des cellules COS-7 avec 50 groupes de 50 plasmides recombinés extraits à partir de doublons positifs a été réalisé. Finalement, un troisième criblage dans des cellules COS-7 a conduits à l'isolation de 2 clones d'ADNc identiques (clones d'ADNc 2C2 et 3G7) qui transférent l'expression de l'antigène dans des cellules COS-7 HLA-B7⁺. Les résultats obtenus avec ces clones d'ADNc sont présentés sur la Figure 3A.

La séquence d'ADNc 2C2 a une longueur de 1250 nt et une homologie de 100 % sur les nt 763 à 2009 (les nt étant numérotés à partir du codon non-sens) d'un ADNc récemment identifié, codant pour une carboxyle estérase intestinale putative (31). Pour identifier l'ADNc de iCE de pleine longueur correspondant à la séquence publiée, une RT-PCR a été effectuée en partant d'ARN total extrait à partir d'une lignée de cellules RCC et le produit PCR de 1,6 kb correspondant a été sous-cloné dans le vecteur pcDNAI, puis séquencé. La séquence de nucléotides est identique à la séquence publiée de iCE. Des expériences de co-transfection dans des cellules COS-7 ont montré que l'ADNc de iCE de pleine longueur est capable de conférer une reconnaissance par 3B8.

### Exemple 5 : Identification du peptide antigénique

Afin de délimiter la région nucléotidique minimale codant pour le peptide antigénique, on a obtenu divers ADNc tronqués, correspondant à la région codante de iCE, à partir du clone 2C2 d'ADNc (Figure 4). On a transfecté dans des cellules COS-7 ces fragments d'ADNc sous-clonés dans le vecteur d'expression pcDNAI, conjointement avec le pcDNAI contenant l'ADNc de HLA-B*0702 autologue. Une région codante nucléotidique minimale est située entre les nucléotides 763 et 1033. Pour réduire la séquence nucléotidique codant pour l'antigène, plusieurs ADNc tronqués ont été obtenus par amplification PCR. Ces ADNc tronqués ont été co-transfectés avec l'allèle HLA-B*0702 dans des cellules COS-7. Les cellules COS-7 transfectées avec un fragment allant des nucléotides 763 à 855 sont reconnues par le 3B8 de CTL mais pas celles transfectées par un fragment allant des nucléotides 763 à 834 (Figure 4), indiquant que la région codante peptidique était située entre les nucléotides 763 et 855. Après examen de la séquence d'acides aminés correspondante, tous les nonamères et décamères possibles ont été synthétisés et leur aptitude à rendre des cellules B transformées par EBV autologues sensibles à une lyse par 3B8 a été évaluée. Aucun d'entre eux ne s'est avéré être positif à 10⁻⁴ ou 10⁻⁵ M.

Finalement, une ORF alternative a été trouvée (une phase ouverte de lecture traductionnelle +1 conduisant à une ORF de 453 nt) avec trois ATG sur les positions nt 476, 479 et 803, qui code un nonamère (SPRWWPTCL) dans la région minimale des nt 763-855. Cette séquence nonamère comprend des résidus d'ancrage de HLA-B7 sur les positions 2, 3 et 9. Une lyse semi-maximale de cellules B transformées par EBV était obtenue avec moins de 10⁻⁶ M de ce nonapeptide (Figure 5).

### Exemple 6 : Fixation du peptide de iCE à HLA-B7

Des antigènes peptidiques de fixation à HLA-A2 régulent vers le haut l'expression de molécules HLA-A2 sur des cellules T2 (37). De façon similaire, des cellules T2 transfectées par HLA-B*0702 (38) ont été utilisées pour analyser la capacité de fixation et la stabilité du peptide de iCE (Figure 6). La fixation du peptide de iCE est stable dans le temps à 50 mM pendant au moins 4 h, contrairement au témoin, le peptide HSP70 à restriction HLA-A2 (14).

### Exemple 7 : Distribution tissulaire d'ARNm de iCE

Pour déterminer la détermination tissulaire de messagers de iCE, un Master blot™ d'ARN humain (Clontech, Palo Alto, USA), constitué d'une membrane en nylon sur laquelle des ARN poly(A)+ provenant de 50 tissus humains ont été immobilisés en taches séparées, a été hybridés avec l'ADNc marqué au ³²P du clone 2C2, servant de sonde. L'ARNm de iCE a été détecté dans le foie, le rein, l'intestin grêle, le colon et le coeur, et il était faiblement exprimé dans l'hypophyse, la surrénale, la prostate et l'estomac. Aucun signal n'a été trouvé dans les tissus foetaux, dans la moelle osseuse, dans les leucocytes périphériques, dans le poumon et dans le cerveau. Pour identifier les espèces d'ARNm, un Northern blot a été préparé avec de l'ARN poly(A)+ provenant de diverses lignées de cellules RCC et lignées de cellules rénales non transformées (Figure 7A), ainsi qu'avec de l'ARN total extrait à partir de diverses tumeurs primaires, à savoir des tumeurs rénales, un mélanome, une tumeur de la vessie, un neuroblastome, une tumeur du côlon (Figure 7B). Le blot d'ARN a été hybridé avec une sonde d'ADNc correspondant aux nucléotides 1033-2009 de la séquence de 2C2. Comme le montre la Figure 7A, deux espèces d'ARNm (4,5 kb, 3,5 kb) antérieurement décrites par Schwer et al. (31), ont été détectées dans des lignées de cellules de carcinome RCC ainsi que dans des cellules rénales non transformées. Dans les tumeurs primaires rénales, un produit de transcription d'ARNm unique (3,5 kb) est détectable, tandis qu'aucun produit de transcription de iCE n'a été détecté dans des tumeurs primaires d'histotypes différents (Figure 7B). Bien qu'un produit de transcription additionnel de 2,2 kb ait été indiqué (31) dans l'intestin grêle et le foie, aucun tel produit de transcription n'a été détecté dans les diverses lignées cellulaires ou tumeurs primaires testées. Ainsi, la protéine de iCE est codée dans les tumeurs RCC à partir d'une espèce d'ARNm unique exprimé de façon prédominante (3,5 kb).

### Exemple 8 : Un codon non-AUG cryptique initie une phase ouverte de lecture alternative

Un codon de terminaison a d'abord été introduit en position 807 de l'ADNc de iCE de pleine longueur (Figure 8A) juste avant la séquence codante nonamère pour confirmer que le peptide reconnu dans les essais de cytotoxicité est codé par la séquence correspondante dans des essais de transfection de COS-7. Cette mutation ponctuelle (mutant A) abolit la reconnaissance de 3B8 de CTL après co-transfection avec HLA-B*0702 dans des cellules COS (8B). Le site d'initiation de traduction AUG naturel a été muté en position 3 et ce mutant ponctuel (mutant B) s'avère être encore reconnu (Figure 8), indiquant que ni la séquence en acide aminé de iCE naturelle ni une séquence chimère résultant d'un décalage de phase traductionnel programmé (c'est-à-dire d'un glissement dans iCE du ribosome d'un codon vers l'avant) et d'un recodage de la séquence en aval (39, 40) ne code le peptide reconnu.

En plus du décalage de phase ribosomal (41), un mécanisme de balayage ribosomal qui initie la traduction au niveau d'un ATG en aval s'est avéré conduire à la production de phases de lecture alternatives reconnues par des cellules T (42). Des mutations ponctuelles sur l'ADNc de iCE de pleine longueur ont été introduites au niveau de chacun des trois sites ATG trouvés dans la ORF +1 en amont du peptide nonamère (mutant C pour les positions 476 et 479 et mutant D en position 803) pour tester si les hybrides pcDNAI de iCE mutés correspondants étaient toujours capables de conférer une reconnaissance par 3B8 de CTL dans des essais de libération de TNF quand ils étaient co-transfectés avec HLA-B*0702 dans des cellules COS. Comme le montre la Figure 8B, aucune de ces mutations n'abolit la reconnaissance par 3B8 de CTL. Ces résultats démontrent qu'un codon non-AUG cryptique est utilisé dans l'ADNc de iCE en tant que site d'initiation de traduction alternatif.

Pour délimiter la région nucléotidique minimale codant pour ce codon non-ATG cryptique, des codons de terminaisons devant interrompre la ORF +1 ont été introduits en différentes positions en amont du peptide antigénique (entre les positions 428 et 809), avec des mutations ponctuelles sur les positions 466 (mutant E), 519, 666 et 786 de l'ADNc de iCE de pleine longueur (Figure 8A). Ces quatre mutants abolissent tous la reconnaissance de 3B8 de CTL après co-transfection (voir sur la Figure 8B le résultat du mutant E pour la position 446). Une région nucléotidique minimale a ensuite été localisée entre les nt 428 et 466. Des codons non-ATG possibles ont ensuite été recherchés (CTG, ACG) dans cette séquence courte, et un codon ACG en position 440 a été trouvé. Une mutation de ce codon en ACT (mutant F) abolit la reconnaissance de 3B8 de CTL (Figure 8B). Ainsi, le premier codon non-AUG dans la ORF +1 a été utilisé pour initier le processus de traduction.

### REFERENCES

1. Van der Bruggen, P., C. Traversari, P. Chomez, C. Lurquin, E. D. Plaen, B. V. d. Eynde, A. Knuth, and T. Boon. 1991. A gene encoding an antigen recognized by cytolytic T lymphocytes on a human melanoma. Science 254:1643.
2. Van den Eynde, B. , and V. G. Brichard. 1995. New tumor antigens recognized by T cells. Curr. Opin. Immunol. 7:674.
3. Gaugler, B., B. V. d. Eynde, P. Van den Bruggen, P. Romero, J. J. Gaforio, E. D. Plaen, B. Lethe, F. Brasseur, and T. Boon. 1994. Human gene MAGE-3 codes for an antigen recognized on a melanoma by autologous cytolytic T lymphocytes. J. Exp. Med. 179:921.
4. Boel, P., C. Wildmann, M. L. Sensi, R. Brasseur, J. C. Renauld, P. Coulie, T. Boon, and P.Van den Bruggen. 1995. BAGE : a new gene encoding an antigen recognized on human melanomas by cytolytic T lymphocytes. Immunity 2:167.
5. Coulie, P. G., V. Brichard, A. V. Pel, T. Wölfel, J. Schneider, C. Traversari, S. Mattei, E. D. Plaen, C. Lurquin, J. P. Szikora, J. C. Renauld, and T. Boon. 1994. A new gene coding for a differentiation antigen recognized by autologous cytolytic T lymphocytes on HLA-A2 melanomas. J. Exp. Med. 180:35.
6. Kawakami, Y., S. Eliyahu, K. Sakaguchi, P. F. Robbins, L. Rivoltini, J. R. Yannelli, E. Appella, and S. A. Rosenberg. 1994. Identification of the immunodominant peptides of the MART-1 human melanoma antigen recognized by the majority of HLA-A2-restricted tumor infiltrating lymphocytes. J. Exp. Med. 180:347.
7. Brichard, V., A. V. Pel, T. Wölfel, C. Wölfel, E. D. Plaen, B. Lethé, P. Coulie, and T. Boon. 1993. The tyrosinase gene codes for an antigen recognized by autologous cytolytic T lymphocytes on HLA-A2 melanomas. J. Exp. Med. 178:489.
8. Robbins, P. F., M.El-Gamil, Y. F. Li, S. L. Topalian, L. Rivoltini, K. Sakaguchi, E. Appella, Y. Kawakami, and S. A. Rosenberg. 1995. Cloning of a new gene encoding an antigen recognized by melanoma-specific HLA-A24-restricted tumor-infiltrating lymphocytes. J. Immunol. 154:5944.
9. Wang, R. F., P. F. Robbins, Y. Kawakami, X. Q. Kang, and S. A. Rosenberg. 1995. Identification of a gene encoding a melanoma tumor antigen recognized by HLA-A31-restricted tumor-infiltrating lymphocytes. J. Exp. Med. 181:799.
10. Robbins, P. F., M. El-Gamil, Y. F. Li, Y. Kawakami, D. Loftus, E. Appella, and S. A. Rosenberg. 1996. A mutated beta-catenin gene encodes a melanoma-specific antigen recognized by tumor infiltrating lymphocytes, J. Exp. Med. 183:1185.
11. Coulie, P. G., F. Lehmann, B. Lethe, J. Herman, C. Lurquin, M. Andrawiss, and T. Boon. 1995. A mutated intron sequence codes for an antigenic peptide recognized by cytolytic T lymphocytes on a human melanoma. Proc. Natl. Acad. Sci. USA 92:7976.
12. Wölfel, T., M. Hauer, J. Schneider, M. Serrano, C. Wolfel, E. Klehmann-Hieb, E. D. Plaen, T. Hankeln, K. H. M. z. Buschenfelde, and D. Beach. 1995. A p16ink4a-insensitive CDK4 mutant targeted by cytolytic T lymphocytes in a human melanoma. Science 269:1281.
13. Brandle, D., F. Brasseur, P. Weynants, T. Boon, and B. J. V. d. Eynde. 1996. A mutated HLA-A2 molecule recognized by autologous cytotoxic T lymphocytes on a human renal cell carcinoma. J. Exp. Med. 183:2501.
14. Gaudin, C., F. Kremer, E. Angevin, V. Scott, and F. Triebel. 1999. A HSP70-2 mutaton recognized by cytolytic T lymphocytes on a human renal cell carcinoma. J. Immunol. 162:1730.
15. Guilloux, Y., S. Lucas, V. G. Brichard, A. VanPel, C. Viret, E. D. Plaen, F. Brasseur, B. Lethe, F. Jotereau, and T. Boon. 1996. A peptide recognized by human cytolytic T lymphocytes on HLA-A2 melanoma is encoded by an intron sequence of the N-acetylglucosaminyltransferase V gene. J. Exp. Med. 183:1173.
16. Robbins, P. F., M. El-Gamil, Y. F. Li, E. B. Fitzgerald, Y. Kawakami, and S. A. Rosenberg. 1997. The intronic region of an incompletely spliced gp100 gene transcript encodes an epitope recognized by melanoma-reactive tumor-infiltrating lymphocytes. J. Immunol. 159.303.
17. Wang, R. F., E. Appella, Y. Kawakami, X. Kang, and S. A. Rosenberg. 1996. Identification of TRP-2 as a human tumor antigen recognized by cytotoxic T lymphocytes. J. Exp. Med. 184:2207.
18. Wang, R. F., S. L. Johnston, G. Zeng, S. L. Topalian, D. J. Schwartzentruber, and S. A. Rosenberg. 1998. A breast and melanoma-shared tumor antigen: T cell responses to antigenic peptides translated from different open reading frames. J Immunol 161:3598.
19. Nanbru, C., I. Lafon, S. Audigier, M. C. Gensac, S. Vagner, G. Huez, and A. C. Prats. 1997. Alternative translation of the proto-oncogene c-myc by an internal ribosome entry site. J. Biol. Chem. 272:32061.
20. Vagner, S., C. Touriol, B. Galy, S. Audigier, M. C. Gensac, F. Almaric, F. Bayard, H. Prats, and A. C. Prats. 1996. Translation of CUG- but not AUG-initiated forms of human fibroblast growth factor 2 is activated in transformed and stressed cells. J. Cell. Biol. 135:1391.
21. Galy, B., A. Maret, A. C. Prats, and H. Prats. 1999. Cell transformation results in the loss of the density-dependent translational regulation of the expression of fibroblast growth factor 2 isoforms. Cancer Res. 59:165.
22. Angevin, E., F.Kremer, C.Gaudin, T.Hercend, and F.Triebel. 1997. Analysis of T-cell immune response in renal cell carcinoma: polarization to type 1-like differentiation pattren, clonal T cell expansion and tumor-specific cytotoxicity. Int. J. Cancer 72:431.
23. Seed, B. 1987. An LFA-3 cDNA encodes a phospholipid-linked membrane protein homologous to its receptor CD2. Nature 329:84O.
24. Traversari, C., P. Van der Bruggen, I. F. Luescher, C. Lurquin, P. Chomez, A. Van Pel, E. De Plaen, A. Amar-Costesec, and T. Boon. 1992. A nonapeptide encoded by human gene MAGE-1 is recognized on HLA-A1 by cytolytic T lymphocytes directed against tumor antigen MZ2-E. J. Exp. Med 176:1453.
25. Espevik, T., and J. Nissen-Meyer. 1986. A highly sensitive cell line, WEHI 164 clone 13, for measuring cytotoxic factor/tumor necrosis factor from human monocytes. J. Immunol. Methods 95:99.
26. Genevee, C., A. Diu, J. Nierat, A. Caignard, P. Y. Dietrich, L. Ferradini, S. Roman-Roman, F. Triebel, and T. Hercend. 1992. An experimentally validated panel of subfamily-specific oligonucleotide primers (Va 1-w29/Vb 1-w24) for the study of human T cell receptor variable V gene segment usage by polymerase chain reaction. Eur. J. Immunol 22:1261.
27. Even, J., A. Lim, I. Puisieux, L. Ferradini, P. Y. Dietrich, A. Toubert, T. Hercend, F. Triebel, C. Pannetier, and P. Kourilsky. 1995. T-cell repertoires in healthy and diseased human tissues analysed by T-cell receptor b -chain CDR3 size determination : evidence for oligoclonal expansions in tumours and inflammatory diseases. Res. Immunol. 146:65.
28. Pannetier, C., M. Cochet, S. Darche, A. Casrouge, M. Zoller, and P. Kourilsky. 1993. The sizes of the CDR3 hypervariable regions of the murine T-cell receptor b chains vary as a function of the recombined germ-line segments. Proc. Natl. Acad. Sci. USA 90:2472.
29. Birnboim, H. C., and J. Doly. 1979. A rapid alkaline extraction procedure for screening recombinant plasmid DNA. Nucleic Acid Res 7:1513.
30. Sambrook, J., E. Fritsch, and T. Maniatis. 1989. Molecular cloning : a laboratory manual. Cold Spring Harbor Laboratory Press, Cold Spring Harbor, NY.
31. Schwer, H., T. Langmann, R Daig, A. Becker, C. Aslanidis, and G. Schmitz. 1997. Molecular cloning and characterization of a novel putative carboxylesterase, present in human intestine and liver. Biochem. Biophys. Res. Comm. 233:117.
32. Mikaelian, I., and A. Sergeant. 1992. A general and fast method to generate multiple site directed mutations. Nuc. Ac. Res. 20:376.
33. Ennis, P. D., J. Zemmour, R. D. Salter, and P. Parham. 1990. Rapid cloning of HLA-A,B cDNA by using the polymerase chain reaction: Frequency and nature of errors produced in amplification. Proc. Natl. Acad. Sci.USA 87:2833.
34. Farace, F., F. Orlanducci, P. Y. Dietrich, C. Gaudin, E. Angevin, M. H. Courtier, C. Bayle, T. Hercend, and F. Triebel. 1994. T cell repertoire in patients with B-chronic lymphocytic leukemia : evidence for multiple in vivo T cell clonal expansions. J. Immunol. 153:4281.
35. Farace, F., E. Angevin, I. Poullion, C. Leboullaire, G. Ferir, D. Elias, B. Escudier, and F. Triebel. 1997. T-cell receptor CDR3 size distribution analysis to evaluate specific T-cell response to cancer vaccines. Int. J. Cancer 71:972.
36. Gaudin, C., P. Y. Dietrich, S. Robache, M. Guillard, B. Escudier, M. J. Terrier-Lacombe, A. Kumar, F. Triebel, and A. Caignard. 1995. In vivo local expansion of clonal T cell subpopulations in renal cell carcinoma. Cancer Res. 55:685.
37. Nijman, H. W., J. G. Houbiers, M. P. Vierboom, S. H. v. d. Burg, J. W. Drijfhout, J. D'Amaro, P. Kenemans, C. J. Melief, and W. M. Kast. 1993. Identification of peptide sequences that potentially trigger HLA-A2. 1-restricted cytotoxic T lymphocytes. Eur. J. Immunol. 23:1215.
38. Smith, K. D., and C. T. Lutz. 1996. Peptide-dependent expression of HLA-B7 on antigen processing deficient T2 cells. J. Immunol. 156:3755.
39. Farabaugh, P. J. 1996. Programmed translational frameshifting. Annu. Rev. Genet. 30:507.
40. Matsufuji, S., T. Matsufuji, Y. Miyazaki, Y. Murakami, J. F. Atkins, R. F. Gesteland, and S. Hayashi. 1995. Autoregulatory frameshifting in decoding mammalian ornithine decarboxylase antizyme. Cell 80:51.
41. Elliott, T., H. Bodmer, and A. Townsend. 1996. Recognition of out-of-frame major histocompatibility complex class I-restricted epitopes in vivo. Eur J Immunol 26:1175.
42. Bullock, T. N. J., and L. C. Eisenlohr. 1996. Ribosomal scanning past the primary initiation codon as a mechanism for expression of CTL epitopes encoded in alternative reading frames. J. Exp. Med. 184:1319.

## Revendications

1. Composé peptidique **caractérisé en ce qu'**il comprend la séquence SEQ ID N°1, ou une partie de la séquence SEQ ID N°1, ladite partie comprenant au moins la séquence SPRWWPTCL (SEQ ID N°2); et ce qu'il entraîne une réponse T spécifique.

2. Composé peptidique selon la revendication 1 ayant la séquence SPRWWPTCL (SEQ ID N°2).

3. Composé peptidique selon l'une des revendications 1 et 2 **caractérisé en ce qu'**il comporte au moins un élément différent des acides aminés naturels.

4. Fragment d'ADN codant pour le peptide de séquence SPRWWPTCL (SEQ ID N°2).

5. Fragment d'ADN de séquence SEQ ID N°3.

6. Cellules dendritiques chargées en composés peptidiques selon l'une des revendications 1 à 2.

7. Cellules dendritiques selon la revendication 6 **caractérisées en ce qu'**elles font parties des macrophages.

8. Composition pharmaceutique comprenant un composé peptidique ou un mélange de composés peptidiques selon l'une des revendications 1 à 2 et un véhicule pharmaceutiquement acceptable.

9. Composition pharmaceutique comprenant notamment un fragment d'ADN selon la revendication 4 ou 5 et un véhicule pharmaceutiquement acceptable.

10. Composition pharmaceutique comprenant les cellules selon l'une des revendications 6 à 7 et un véhicule pharmaceutiquement acceptable.

11. Composition pharmaceutique selon l'une des revendications 8 à 10 **caractérisée en ce qu'**il comporte en outre un ou plusieurs adjuvents immunologiques, notamment des agents cytotoxiques des tumeurs.

12. Composition pharmaceutique selon l'une des revendications 8 à 11 **caractérisée en qu'**il comporte un véhicule pharmaceutique compatible avec une administration IV, subcutanée, orale, ou nasale.

13. Composition pharmaceutique selon l'une des revendications 8 à 12 **caractérisée en ce qu'**il comporte un véhicule pharmaceutique sélectionné parmi les liposomes, les nano-particules, ou les émulsions lipidiques, chargés positivement ou négativement.

14. Utilisation d'un composé peptidique selon l'une des revendications 1 à 2 pour la fabrication d'un médicament.

15. Utilisation d'un composé peptidique selon l'une des revendications 1 à 2 pour la fabrication d'un médicament destiné au traitement du cancer.

16. Utilisation d'un composé peptidique selon l'une des revendications 1 à 2 pour la fabrication d'un médicament destiné à une immunisation ex vivo, consistant notamment à induire in vitro des CTL spécifiques de tumeurs, les expandre et les réinjectés, ladite induction pouvant être effectuée à l'aide de cellules dendritiques chargées.

17. Utilisation d'un composé peptidique selon l'une des revendications 1 à 2 pour la fabrication d'un médicament destiné à une immunisation in vivo.

18. Utilisation d'un composé peptidique selon l'une des revendications 1 à 2 pour la fabrication d'un médicament destiné au traitement du cancer, en particulier des tumeurs solides, notamment des carcinomes, des mélanomes, des neuroblastomes, de préférence des hépatocarcinomes et des adénocarcinomes du côlon ou du rein.

19. Utilisation d'un composé peptidique selon l'une des revendications 1 à 2 pour augmenter en milieu de culture la population des CTL des tumeurs et/ou induire la sécrétion par lesdits CTL de facteurs cytotoxiques tels que par exemple IL-2, IFN-γ et TNF.

20. Utilisation d'un composé peptidique selon l'une des revendications 1 à 2 pour la fabrication d'un médicament destiné à stimuler les défenses immunitaires, en particulier pour augmenter la population des CTL des tumeurs et/ou induire la sécrétion par lesdits CTL de facteurs cytotoxiques tels que par exemple IL-2, IFN-γ et TNF.

21. Anticorps monoclonal se liant au composé peptidique ayant la séquence SEQ ID No 1 dans un essai immunologique.

22. Procédé de détection d'un peptide ou polypeptide codé par l'ORF+1 de iCE, comprenant les étapes consistant à :
a) mettre en contact un échantillon prélevé chez un individu avec un anticorps monoclonal selon la revendication 21,
b) permettre la formation du complexe anticorps-peptide ou polypeptide,
a) détecter ledit peptide ou polypeptide au moyen d'un marqueur détectable dans le complexe ou qui se lie au complexe.

23. Trousse de diagnostic comportant notamment un anticorps selon la revendication 21 pour la détection du cancer.

24. Trousse de diagnostic comportant notamment un anticorps selon la revendication 21 pour le prognostique du cancer déclaré chez un individu.

25. Composition pharmaceutique comprenant notamment un anticorps monoclonal selon la revendication 21 et un véhicule pharmaceutiquement acceptable.

## Patentansprüche

1. Peptidverbindung, **dadurch gekennzeichnet, dass** sie die Sequenz SEQ ID Nr. 1 oder einen Teil der Sequenz SEQ ID Nr. 1, wobei der Teil wenigstens die Sequenz SPRWWPTCL (SEQ ID Nr. 2) umfasst, umfasst und dass sie eine T-spezifische Antwort auslöst.

2. Peptidverbindung nach Anspruch 1, welche die Sequenz SPRWWPTCL (SEQ ID Nr. 2) aufweist.

3. Peptidverbindung nach einem der Ansprüche 1 und 2, **dadurch gekennzeichnet, dass** sie wenigstens ein Element, welches von den natürlichen Aminosäuren verschieden ist, umfasst.

4. DNA-Fragment, welches das Peptid mit der Sequenz SPRWWPTCL (SEQ ID Nr. 2) kodiert.

5. DNA-Fragment mit der Sequenz SEQ ID Nr. 3.

6. Dendritische Zellen, die mit Peptidverbindungen nach einem der Ansprüche 1 bis 2 beladen sind.

7. Dendritische Zellen nach Anspruch 6, **dadurch gekennzeichnet, dass** sie zu den Makrophagen gehören.

8. Pharmazeutische Zusammensetzung, welche eine Peptidverbindung oder eine Mischung von Peptidverbindungen nach einem der Ansprüche 1 bis 2 und einen pharmazeutisch annehmbaren Träger umfasst.

9. Pharmazeutische Zusammensetzung, welche insbesondere ein DNA-Fragment nach Anspruch 4 oder 5 und einen pharmazeutisch annehmbaren Träger umfasst.

10. Pharmazeutische Zusammensetzung, welche die Zellen nach einem der Ansprüche 6 bis 7 und einen pharmazeutisch annehmbaren Träger umfasst.

11. Pharmazeutische Zusammensetzung nach einem der Ansprüche 8 bis 10, **dadurch gekennzeichnet, dass** sie außerdem eine oder mehrere immunologische Hilfssubstanzen, insbesondere für Tumore zytotoxische Mittel, umfasst.

12. Pharmazeutische Zusammensetzung nach einem der Ansprüche 8 bis 11, **dadurch gekennzeichnet, dass** sie einen pharmazeutischen Träger, welcher mit einer intravenösen, subkutanen, oralen oder nasalen Verabreichung verträglich ist, umfasst.

13. Pharmazeutische Zusammensetzung nach einem der Ansprüche 8 bis 12, **dadurch gekennzeichnet, dass** sie einen pharmazeutischen Träger, ausgewählt unter den Liposomen, den Nanopartikeln oder den lipidischen Emulsionen, die positiv oder negativ geladen sind, umfasst.

14. Verwendung einer Peptidverbindung nach einem der Ansprüche 1 bis 2 für die Herstellung eines Arzneimittels.

15. Verwendung einer Peptidverbindung nach einem der Ansprüche 1 bis 2 für die Herstellung eines Arzneimittels, welches für die Behandlung von Krebs bestimmt ist.

16. Verwendung einer Peptidverbindung nach einem der Ansprüche 1 bis 2 für die Herstellung eines Arzneimittels, welches für eine Immunisierung ex vivo bestimmt ist, welche insbesondere darin besteht, Tumor-spezifische zytotoxische T-Lymphozyten in vitro zu induzieren, diese zu vermehren und diese erneut zu injizieren, wobei die Induktion mit Hilfe von beladenen dendritischen Zellen ausgeführt werden kann.

17. Verwendung einer Peptidverbindung nach einem der Ansprüche 1 bis 2 für die Herstellung eines Arzneimittels, welches für eine Immunisierung in vivo bestimmt ist.

18. Verwendung einer Peptidverbindung nach einem der Ansprüche 1 bis 2 für die Herstellung eines Arzneimittels, welches für die Behandlung von Krebs, insbesondere von soliden Tumoren, insbesondere von Karzinomen, Melanomen, Neuroblastomen, vorzugsweise Leberzellkarzinomen und Adenokarzinomen des Kolons oder der Niere, bestimmt ist.

19. Verwendung einer Peptidverbindung nach einem der Ansprüche 1 bis 2 zur Erhöhung der Population der für Tumore zytotoxischen T-Lymphozyten in Kulturmedium und/oder zur Induktion der Sekretion von zytotoxischen Faktoren, wie beispielsweise IL-2, IFN-γ und TNF, durch die zytotoxischen T-Lymphozyten.

20. Verwendung einer Peptidverbindung nach einem der Ansprüche 1 bis 2 zur Herstellung eines Arzneimittels, welches dazu bestimmt ist, die Immunabwehren zu stimulieren, insbesondere zur Erhöhung der Population der für Tumore zytotoxischen T-Lymphozyten und/oder zur Induktion der Sekretion von zytotoxischen Faktoren, wie beispielsweise IL-2, IFN-γ und TNF, durch die zytotoxischen T-Lymphozyten.

21. Monoklonaler Antikörper, der in einem immunologischen Assay an die Peptidverbindung mit der Sequenz SEQ ID Nr. 1 bindet.

22. Verfahren zur Detektion eines Peptids oder Polypeptids, welches durch das ORF+1 von iCE kodiert wird, welches die Schritte umfasst, welche darin bestehen:
a) eine von einem Individuum entnommene Probe mit einem monoklonalen Antikörper nach Anspruch 21 in Kontakt zu bringen,
b) die Bildung des Antikörper-Peptid- oder -Polypeptid-Komplexes zu erlauben,
a) das Peptid oder Polypeptid mittels eines detektierbaren Markers in dem Komplex oder der sich an den Komplex bindet, zu detektieren.

23. Diagnostischer Kit, welcher insbesondere einen Antikörper nach Anspruch 21 für die Detektion von Krebs umfasst.

24. Diagnostischer Kit, welcher insbesondere einen Antikörper nach Anspruch 21 für die Prognose der bei einem Individuum ausgebrochenen Krebserkrankung umfasst.

25. Pharmazeutische Zusammensetzung, welche insbesondere einen monoklonalen Antikörper nach Anspruch 21 und einen pharmazeutisch annehmbaren Träger umfasst.

## Claims

1. Peptide compound, **characterized in that** it comprises the sequence SEQ ID No. 1 or a part of the sequence SEQ ID No. 1, the said part comprising at least the sequence SPRWWPTCL (SEQ ID No. 2), and **in that** it causes a specific T response.

2. Peptide compound according to Claim 1, having the sequence SPRWWPTCL (SEQ ID No. 2).

3. Peptide compound according to either of Claims 1 and 2, **characterized in that** it comprises at least one element other than natural amino acids.

4. DNA fragment encoding the peptide of sequence SPRWWPTCL (SEQ ID No. 2).

5. DNA fragment of sequence SEQ ID No. 3.

6. Dendritic cells loaded with peptide compounds according to either of Claims 1 and 2.

7. Dendritic cells according to Claim 6, **characterized in that** they form part of the macrophages.

8. Pharmaceutical composition comprising a peptide compound or a mixture of peptide compounds according to either of Claims 1 and 2 and a pharmaceutically acceptable vehicle.

9. Pharmaceutical composition comprising in particular a DNA fragment according to Claim 4 or 5 and a pharmaceutically acceptable vehicle.

10. Pharmaceutical composition comprising the cells according to either of Claims 6 and 7 and a pharmaceutically acceptable vehicle.

11. Pharmaceutical composition according to one of Claims 8 to 10, **characterized in that** it also comprises one or more immunological adjuvants, in particular agents which are cytotoxic for tumours.

12. Pharmaceutical composition according to one of Claims 8 to 11, **characterized in that** it comprises a pharmaceutical vehicle which is compatible with IV, subcutaneous, oral or nasal administration.

13. Pharmaceutical composition according to one of Claims 8 to 12, **characterized in that** it comprises a pharmaceutical vehicle selected from positively or negatively charged liposomes, nanoparticles or lipid emulsions.

14. Use of a peptide compound according to either of Claims 1 and 2 for manufacturing a medicinal product.

15. Use of a peptide compound according to either of Claims 1 and 2 for manufacturing a medicinal product intended for treating cancer.

16. Use of a peptide compound according to either of Claims 1 and 2 for manufacturing a medicinal product intended for immunization ex vivo, which consists in particular in inducing tumour-specific CTLs in vitro, expanding them and reinjecting them, the said induction possibly being carried out with the aid of loaded dendritic cells.

17. Use of a peptide compound according to either of Claims 1 and 2 for manufacturing a medicinal product intended for immunization in vivo.

18. Use of a peptide compound according to either of Claims 1 and 2 for manufacturing a medicinal product intended for the treatment of cancer, in particular solid tumours, especially carcinomas, melanomas, neuroblastomas, preferably hepatocarcinomas and adenocarcinomas of the colon or of the kidney.

19. Use of a peptide compound according to either of Claims 1 and 2 for increasing, in culture medium, the CTL population of tumours and/or inducing the secretion by the said CTLs of cytotoxic factors such as, for example, IL-2, IFN-γ and TNF.

20. Use of a peptide compound according to either of Claims 1 and 2 for manufacturing a medicinal product intended for stimulating immune defences, in particular to increase the CTL population of tumours and/or to induce the secretion by the said CTLs of cytotoxic factors such as, for example, IL-2, IFN-γ and TNF.

21. Monoclonal antibody which binds to the peptide compound having the sequence SEQ ID No. 1 in an immunological assay.

22. Method for detecting a peptide or polypeptide encoded by the ORF+1 of iCE, comprising the steps consisting in:
a) bringing a sample removed from an individual into contact with a monoclonal antibody according to Claim 21,
b) allowing the formation of the peptide or polypeptide/antibody complex,
c) detecting the said peptide or polypeptide by means of a detectable label which is in the complex or which binds to the complex.

23. Diagnostic kit comprising in particular an antibody according to Claim 21 for detecting cancer.

24. Diagnostic kit comprising in particular an antibody according to Claim 21 for the prognostic of existing cancer in an individual.

25. Pharmaceutical composition comprising in particular a monoclonal antibody according to Claim 21 and a pharmaceutically acceptable vehicle.


### LISTAGE DE SEQUENCES

<110> INSTITUT GUSTAVE ROUSSY
<120> COMPOSE PEPTIDIQUE DERIVE D'UNE ORF DECALEE DU GENE iCE
<130> D18280
<160> 5
<170> PatentIn Vers. 2.0
<210> 1
   <211> 162
   <212> PRT
   <213> Homo sapiens
<220>
   <223> Polypeptide codé par la phase ouverte de lecture alternative et décalée (+1) de iCE humain
<400> 1
   Thr Val Val Arg Leu Phe Leu Ala Trp Leu Pro Cys Met Met Val Pro
   1 5 10 15
   Cys Trp Leu Pro Trp Arg Thr Trp Trp Trp Ser Ser Ser Ser Thr Ala
   20 25 30
   Trp Val Ser Trp Ala Ser Ser Ala Leu Glu Thr Ser Thr Gln Pro Ala
   35 40 45
   Thr Gly Ala Thr Trp Thr Lys Trp Leu His Tyr Ala Gly Ser Ser Arg
   50 55 60
   Ile Ser Pro Thr Leu Glu Ala Thr Leu Thr Val Ser Pro Phe Leu Ala
   65 70 75 80
   Ser Leu Arg Val Ala Arg Val Cys Leu Arg Leu Leu Cys Pro Pro Tyr
   85 90 95
   Pro Lys Asp Ser Ser Thr Glu Pro Ser Trp Arg Val Ala Trp Pro Ser
   100 105 110
   Cys Pro Ala Ser Leu Pro Ala Gln Leu Met Ser Ser Pro Arg Trp Trp
   115 120 125
   Pro Thr Cys Leu Pro Val Thr Lys Leu Thr Leu Arg Pro Trp Trp Ala
   130 135 140
   Ala Cys Gly Ala Arg Val Lys Arg Arg Phe Leu Gln Leu Thr Ser Leu
   145 150 155 160
   Ser Arg
<210> 2
   <211> 9
   <212> PRT
   <213> Homo sapiens
<220>
   <223> Fragment peptidique provenant de la SEQ ID n° 1 entraînant une réponse T spécifique
<400> 2
   Ser Pro Arg Trp Trp Pro Thr Cys Leu
   1 5
<210> 3
   <211> 521
   <212> ADN
   <213> Homo sapiens
<220>
   <223> Phase ouverte de lecture alternative et décalée (+1) de iCE
<400> 3
   acggtggtgc gcttgttttt ggcatggctt ccttgtatga tggttccatg ctggctgcct 60
   tggagaacgt ggtggtggtc atcatccagt accgcctggg tgtcctgggc ttcttcagca 120
   ctggagacaa gcacgcaacc ggcaactggg gctacctgga ccaagtggct gcactacgct 180
   gggtccagca gaatatcgcc cactttggag gcaaccctga ccgtgtcacc atttttggcg 240
   agtctgcggg tggcacgagt gtgtcttcgc ttgttgtgtc ccccatatcc caaggactct 300
   tccacggagc catcatggag agtggcgtgg ccctcctgcc cggcctcatt gccagctcag 360
   ctgatgtcat ctccacggtg gtggccaacc tgtctgcctg tgaccaagtt gactctgagg 420
   ccctggtggg ctgcctgcgg ggcaagagta aagaggagat tcttgcaatt aacaagcctt 480
   tcaagatgat ccccggagtg gtggatgggg tcttcctgcc c                     521
<210> 4
   <211> 30
   <212> ADN
   <213> Homo sapiens
<220>
   <223> Amorce P1
<220>
   <223> Sens
<400> 4
   cccaagcttg gtgaatagca gcgtgtccgc                                  30
<210> 5
   <211> 30
   <212> ADN
   <213> Homo sapiens
<220>
   <223> Amorce P2
<220>
   <223> Antisens
<400> 5
   tgctctagaa gggagctaca gctctgtgtg